(19) Europäisches Patentamt
European Patent Office
Office européen des brevets

(11) Publication number: 0 085 756
A1

# (12) EUROPEAN PATENT APPLICATION

(21) Application number: 82110269.6

(22) Date of filing: 08.11.82

(51) Int. Cl.$^3$: C 07 C 103/42
C 07 D 233/64
//C07D487/04, (C07D487/04, 235/00, 253/00)

(30) Priority: 10.02.82 US 347653
28.06.82 US 393003

(43) Date of publication of application:
17.08.83 Bulletin 83/33

(84) Designated Contracting States:
AT BE CH DE FR GB IT LI LU NL SE

(71) Applicant: AMERICAN CYANAMID COMPANY
1937 West Main Street P.O. Box 60
Stamford Connecticut 06904(US)

(72) Inventor: Raghu, Sivaraman
c/o American Cyanamid Company P.O. Box 60
Stamford, Conn. 06904(US)

(72) Inventor: Farina, James Salvatore
14 Beechwood Drive
Rouses Point New York 12979(US)

(72) Inventor: Peake, Steven Loyalty
16 East Maple Street
New Canaan Connecticut 06840(US)

(74) Representative: Wächtershäuser, Günter, Dr.
Tal 29
D-8000 München 2(DE)

(54) Process for preparing 1-substituted-6-n-propyl-8-methylimidazo(1,5-d)-as-triazin-4(3H)-ones.

(57) This disclosure describes novel processes for the preparation of 2-(*n*-propyl)-4-methyl-5-(lower alkanoyl) imidazoles which are useful as intermediates for the preparation of anti-asthmatic agents.

EP 0 085 756 A1

28,406

# PROCESS FOR PREPARING 1-SUBSTITUTED-6-n-PROPYL-8-METHYLIMIDAZO[1,5-d]-as-TRIAZIN-4(3H)-ones

This invention relates to novel methods for the preparation of 5-(lower alkanoyl)-4-methyl-2-(n-propyl)-imidazoles (IV) which are the immediate precursors of the corresponding 1-(lower alkyl)-6-(n-propyl)-8-methylimidazo[1,5-d]-as-triazin-4(3H)-ones (VI). These final products of the novel processes of the present invention are highly useful for meliorating asthma and for inhibiting diesterase in mammals. They inhibit the release of mediators (to the extent of 50%) from the human basophil at 13μM concentration and also protect guinea pigs from anaphylactic shock. In the mouse, they are active both orally and intraperitoneally in inhibiting passive cutaneous anaphylaxis.

The original eight step synthesis of the anti-asthamatic agents (VI), as set forth in U.S. Reissue Patent No. 30,511, proceeded through the key intermediates (IV). These intermediates were prepared in six steps, three of which [(a) an exothermic mitric acid oxidation, (b) a Grignard reaction, and (c) an environmentally unacceptable chronic acid oxidation] were potentially hazardous for scale-up. It has now been discovered that the novel synthesis of the present invention proceeds in high yield and is totally free of all scale-up hazards. The steps involved in the novel processes of the present invention may be depicted as follows:

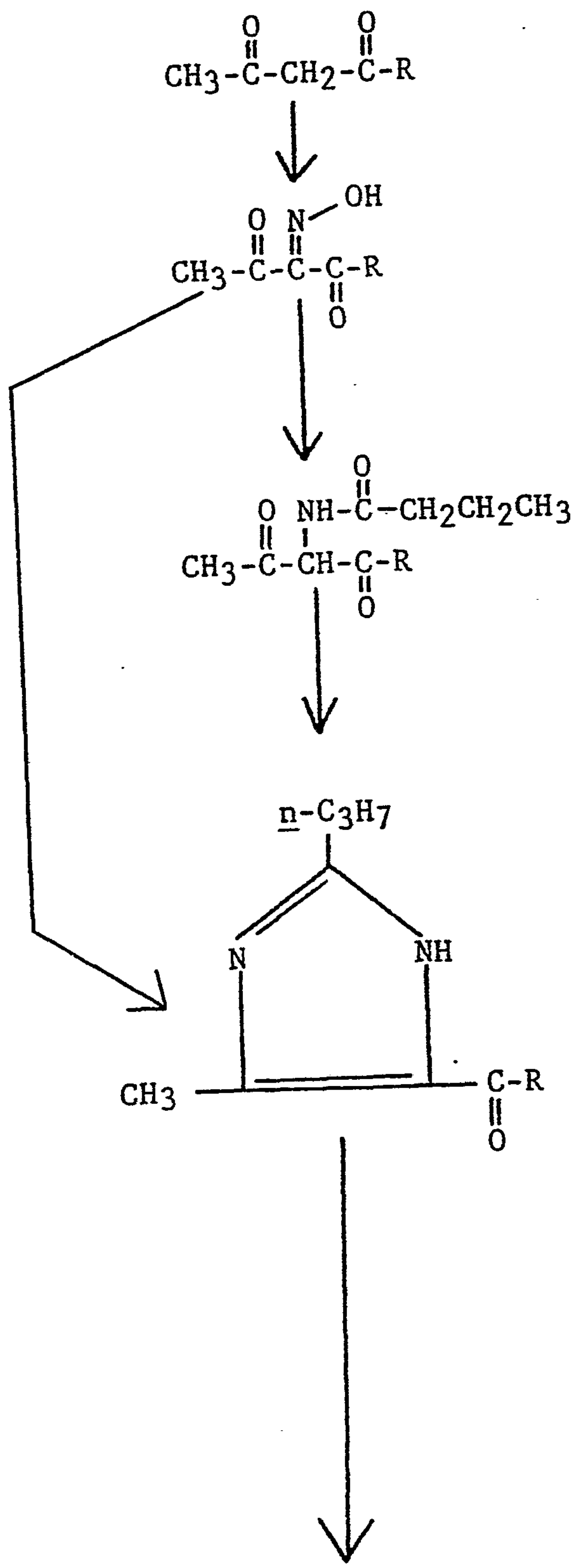

(I)

(II)

(III)

(IV)

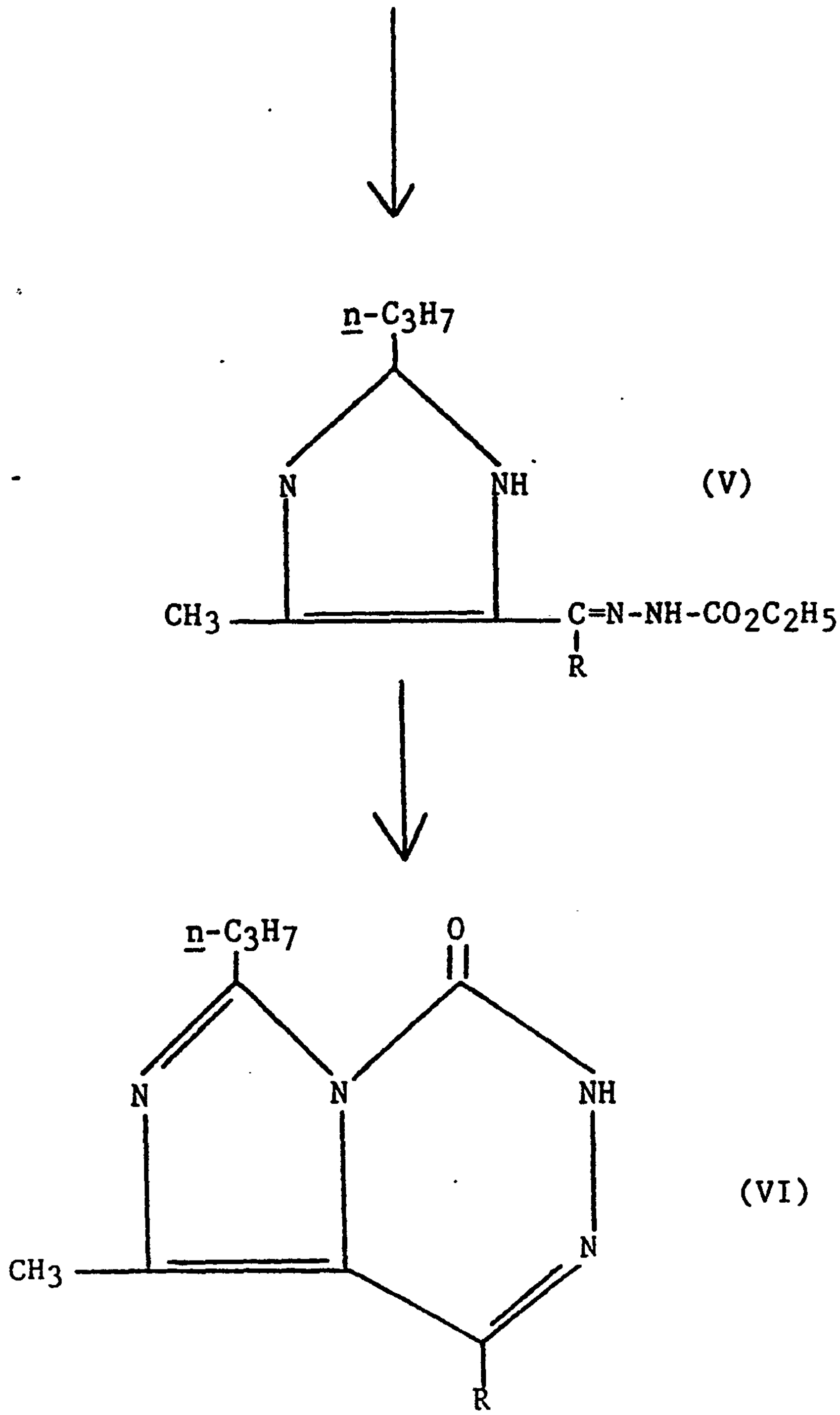

(V)

(VI)

wherein R is methyl, ethyl or n-propyl.

In accordance with the above reaction scheme, treatment of a 2,4-alkanedione (I) with sodium nitrite in aqueous sulfuric acid provides the corresponding 3--oximino-2,4-alkanedione (II) in high yield. The catalytic hydrogenation of (II) with palladium on charcoal as catalyst in the presence of butyric anhydride provides the corresponding 3-butyrylamino-2,4-alkanedione (III) also in high yield. Cyclization of (III) to the corresponding 2-(n-propyl)-4-methyl-5-acylimidazoles (IV) is readily accomplished by heating (III) with a suitable ammoniating agent such as ammonium acetate, ammonium chloride, ammonium sulfate, etc. in an inert organic solvent such as formamide toluene, xylene, chlorobenzene, and the like. Condensation of (II) with commercially available 2-aminovaleric acid or its esters provides (IV) directly. This condensation is conveniently carried out in a suitable inert solvent (e.g., acetonitrile, propionitrile, 2-methoxyethanol, 2-ethoxyethanol, dimethoxyethane, n-butanol DMF, toluene) at the reflux temperature (80°-135°C.) for a period of time of from about two to about 24 hours. Condensation of (IV) with ethyl carbazate in n-butanol or diphenyl ether under reflux for several hours provides (V) which may be isolated by evaporation of the reaction solvent. Cyclization of (V) is accomplished in diphenyl ether at 150°-250°C. for 15-45 minutes to provide the anti-asthmatic agents (VI). Isolation of (VI) is achieved by dilution of the reaction mixture with petroleum ether or by extraction of the reaction mixture with 10% aqueous hydrochloric acid. The acid extract is neutralized with potassium carbonate and the product extracted with solvents such as chloroform or ethyl acetate.

The invention will be described in greater detail in conjunction with the following specific examples.

## Example 1

## Preparation of 3-butyramido-2,4-pentanedione

3-Oximino-2,4-pentanedione (38.7 g., 0.3 mol) was dissolved in a solution of butyric acid (30 ml) and butyric anhydride (150 ml., 0.9 mol) contained in a 500 ml. Parr bottle. The 10% Pd on charcoal catalyst (2 g.) was added and the reaction mixture was purged and pressurized with hydrogen to 55 psi, and shaken at room temperature until the theoretical volume of hydrogen had been consumed. The catalyst was separated by filtration and the butyric acid was removed under vacuum. The resulting oily crystals were recrystallized from hot dibutyl ether to give 49.7 g. of product (89.5% yield) m.p. 74°-75°C.

The above procedure was repeated using the following oximes: 3-oximino-2,4-hexanedione and 3-oximino--2,4-heptandione.

## Example 2

## Preparation of 5-acetyl-4-methyl-2-*n*-propylimidazole

3-Oximino-2,4-pentanedione (1.5 g., 11 mmol) was dissolved in butyic acid (2 ml.) and butyric anhydride (10 ml.) contained in a 250 ml. Parr bottle, then 10% Pd on charcoal catalyst (0.1 g.) was added and the reaction mixture was purged and pressurized with hydrogen to 55 psi. After shaking at room temperature for 3 hours, the theoretical amount of hydrogen had been consumed. The catalyst was removed by filtration and 12 ml. of concentration $NH_4OH$ was added to the filtrate. The reaction mixture was heated to 120°C. for 18 hours, then cooled to room temperature. Saturated sodium chloride solution and $CH_2Cl_2$ were added. The organic phase was washed twice with 20% HCl solution, and the combined acidic phases were neutralized with concentrated $NH_4OH$ and washed with $CH_2Cl_2$. The organic phase was dried and the solvent was evaporated to give 0.5 g. of the imidazole as a slight brown oil.

Using the procedure above, the oximes listed in Example 1 were converted into the corresponding imidazoles. In the case of unsymmetrical oximes, the ratio of product isomers was approximately 1:1.

Example 3

Preparation of 5-acetyl-4-methyl-2-n-propylimidazole

To 40 g. (0.309 moles) of 3-oximino-2,4-pentanedione dissolved in 200 ml. of *n*-butanol was added 39.92 g. (0.341 moles) of d,l-norvaline. The resulting mixture was brought to reflux, while stirring rapidly under nitrogen with an oil bath. After 19.3 h the reaction mixture was cooled, and then suction filtered. The residue was washed with ethyl acetate (100 ml.) and the combined filtrates concentrated *in vacuo*. The resulting oil was dissolved in ethyl acetate (200 ml.) and extracted with three 50 ml. portions of 5% aqeuous sodium hydroxide solution. Removal of the solvent *in vacuo* gave 0.15 moles (55.7% yield based on recovered d,l--norvaline) of 5-acetyl-4-methyl-2-*n*-propylimidazole.

Example 4

Preparation of 5-acetyl-4-methyl-2-*n*-propylimidazole

An acetonitrile (250 ml.) mixture of 3--oximino-2,4-pentanedione (40 g., 0.31 moles) and d,l-norvaline (43.55 g., 0.372 moles) was heated to reflux under nitrogen. After 23.7 h the reaction mixture was allowed to cool, then suction filtered. The residue was washed with acetonitrile and the combined filtrates concentrated *in vacuo*. After dissolving the resulting oil in ethyl acetate (100 ml.), it was washed with three 40 ml. portions of 5% aqueous sodium hydroxide solution. The combined organic extracts were dried over anhydrous potassium carbonate and then concentrated *in vacuo* to give 0.1 moles (43.5% yield based on recovered d,l--norvaline) of 5-acetyl-4-methyl-2-*n*-propylimidazole.

## Example 5

### Preparation of 5-acetyl-4-methyl-2-n-propylimidazole

To 40 g. (0.31 moles) of 3-oximino-2,4-pentanedione dissolved in 297 ml. of acetonitrile was added 40.64 g. (0.31 moles) of methyl d,l-norvaline. The reaction mixture was refluxed 21.2 h under nitrogen, cooled, then concentrated *in vacuo*. The resulting oil was dissolved in 50 ml. of ethanol and 140 ml. of 10% aqueous sodium hydroxide. After 4 h of reflux, the cooled mixture was extracted with ethyl acetate (900 ml. total) and dried over anhydrous sodium sulfate. Removal of the solvent *in vacuo* afforded 0.16 moles (51% yield) of 5-acetyl-4-methyl-2-n-propylimidazole.

## Example 6

### Preparation of 5-propionyl-4-methyl-2-n-propylimidazole

To 92.4 g. (0.646 moles) of 3-oximino-2,4--hexanedione dissolved in 807 ml. of n-butanol was added 90.75 g. (0.775 moles) of d,l-norvaline. The mixture was refluxed 6.25 h, then cooled and concentrated *in vacuo*. The residue was taken up in ethyl acetate (500 ml.), suction filtered and the filtrate washed with 10% aqueous sodium hydroxide (4 x 100 ml.). Removal of the solvent *in vacuo* gave 0.29 moles (48% yield based on recovered amino acid) of 4-methyl-5-propinoyl-2-propylimidazole and 5-acetyl-4-methyl-2-n-propylimidazole (87:13 respectively).

## Example 7

### Preparation of 1,8-dimethyl-6-n-propylimidazo[1,5-d]--as-triazin-4(3H)-one

A mixture of 59.7 g. of methyl 2-n-propyl-4--methyl-5-imidazolyl ketone, 41.14 g. of ethyl carbazate, 200 ml. of n-butanol and 4 drops of glacial acetic acid is warmed until solution is complete and then heated under reflux for 5 hours. The solution is concentrated *in vacuo* to an oily residue, 250 ml. of diphenyl ether are added and the resulting solution is heated with stirring in an oil bath for 30 minutes after the start

of gas evolution. The temperature is maintained as closely as possible to the point at which gas evolution starts (150°-250°C.). The reaction is removed from the oil bath, cooled to 50°C. and diluted with 1-2 volumes hexane. The product is collected, washed with petroleum ether and then dissolved in 200 ml. of chloroform. This solution is filtered through 250 ml. of Magnesol® followed by an 800 ml. chloroform wash. The filtrate is concentrated *in vacuo* and the residue is recrystallized from 250 ml. of ethyl acetate, giving 48.2 g. of the desired product as off-white crystals, m.p. 154°-155°C.

Example 8

Preparation of 1-ethyl-6-*n*-propyl-8-methylimidazo-[1,5-d]-*as*-triazin-4(3H)-one

The procedure of Example 7 was repeated using ethyl 2-*n*-propyl-4-methyl-5-imidazolyl ketone in place of the methyl 2-*n*-propyl-4-methyl-5-imidazolyl ketone of that example whereby the title product, m.p. 147°-150°C., was obtained.

Example 9

Preparation of 1,6-di-*n*-propyl-8-methylimidazo-[1,5-d]-*as*-triazin-4(3H)-one

The procedure of Example 7 was repeated using *n*-propyl 2-n-propyl-4-methyl-5-imidazolyl ketone in place of the methyl 2-n-propyl-4-methyl-5-imidazolyl ketone of that example whereby the title product, m.p. 145°-146°C., was obtained.

Example 10

Preparation of 1,8-dimethyl-6-*n*-propylimidazo[1,5-d]-*as*-triazin-4(3H)-one, monohydrate, monohydrochloride

A 10.0 g. portion of 1,8-dimethyl-6-*n*-propylimidazo[1,5-d]-*as*-triazin-4(3H)-one was dissolved in 800 ml. of dichloromethane and treated with a stream of hydrogen chloride gas over a 30 minute period. The resulting precipitate was collected, slurried with two

60 ml. portions of dichloromethane and then dried *in vacuo*, giving 10.6 g. of the desired product as a white solid, m.p. 249°-255°C.

## Example 11

### Preparation of 1-ethyl-6-*n*-propyl-8-methylimidazo[1,5-d]-*as*-triazin-4(3H)-one, monohydrate, monohydrochloride

The procedure of Example 10 was repeated using 1-ethyl-6-*n*-propyl-8-methylimidazo[1,5-d]-*as*-triazin-4(3H)-one in place of 1,8-dimethyl-6-*n*-propylimidazo-[1,5-d]-*as*-triazin-4(3H)-one and giving the desired product, m.p. 236°-243°C.

## Example 12

### Preparation of 1,6-di-*n*-propyl-8-methylimidazo[1,5-d]-*as*-triazin-4(3H)-one, monohydrate, monohydrochloride

The procedure of Example 10 was repeated using 1,6-di-*n*-propyl-8-methylimidazo[1,5-d]-*as*-triazin-4(3H)-one in place of 1,8-dimethyl-6-*n*-propylimidazo[1,5-d]-*as*-triazin-4(3H)-one and giving the desired product, m.p. 225°C. (dec.).

8,406

## CLAIMS

We claim:

1. The process of preparing 3-butyrylamino--2,4-alkanediones of the formula:

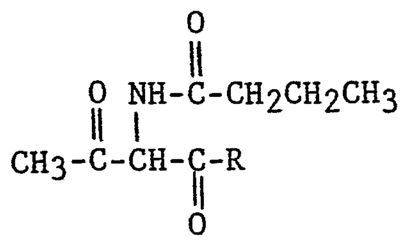

wherein R is methyl, ethyl or n-propyl which comprises hydrogenating a 3-oximino-2,4-alkanedione of the formula:

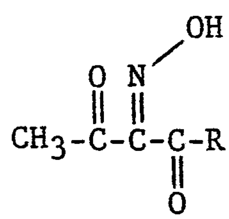

wherein R is as hereinabove defined with palladium on charcoal as catalyst in butyric anhydride as solvent at a temperature fo $25^{o}$-$100^{o}$C. for a period of time of 8-24 hours.

2. The process of preparing 5-imidazolyl ketones of the formula:

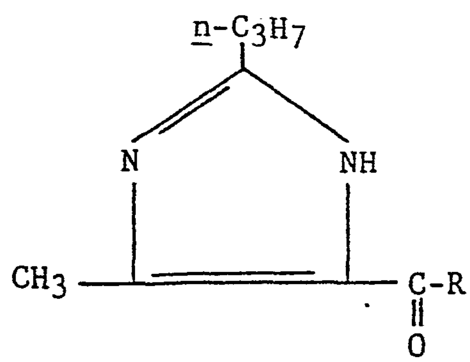

wherein R is methyl, ethyl or n-propyl which comprises condensing a 3-butyrylamino-2,4-alkanedione of the formula:

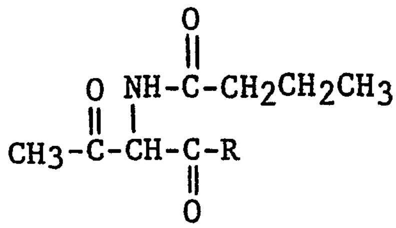

wherein R is as hereinabove defined with an ammoniating agent in an inert organic solvent at a temperature of 100°-150°C. for a period of time of 8-24 hours.

3. The process of preparing 5-imidazolyl ketones of the formula:

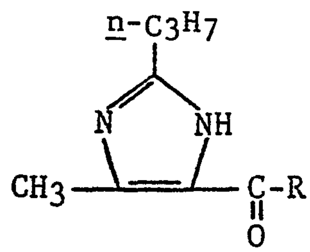

wherein R is methyl, ethyl or n-propyl which comprises condensing a 3-oximino-2,4-alkanedione of the formula:

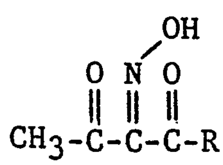

wherein R is as hereinabove defined with *d*,*l*-norvaline or esters thereof in an inert solvent at from about about 80°C. to about 135°C. for a period of time of from about two hours to about 24 hours.

European Patent Office

**0085756**

Application number

EP 82 11 0269

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | JOURNAL OF HETEROCYCLIC CHEMISTRY, vol. 17, December 1980, pages 1723-1726, A.C. VERONESE et al.: "Syntheses of 2-arylimidazole derivatives through annelations employing benzylamines" * Pages 1723-1726 * | 1,2 | C 07 C 103/42<br>C 07 D 233/64 //<br>C 07 D 487/04<br>(C 07 D 487/04<br>C 07 D 235/00<br>C 07 D 253/00 ) |
| Y | DE-A-2 064 520 (CIBA-GEIGY) * Pages 35-43 * | 1,2 | |
| D,Y | US-E- 30 511 (R. PAUL and J. MENSCHIK) * Columns 1-4 * | 1,2 | |
| P,Y | EP-A-0 057 289 (AMERICAN CYANAMID) * Page 2, lines 12-14 * | 1,2 | TECHNICAL FIELDS SEARCHED (Int. Cl. 3)<br>C 07 C 103/00<br>C 07 D 233/00<br>C 07 D 487/00 |

The present search report has been drawn up for all claims

| Place of search | Date of completion of the search | Examiner |
|---|---|---|
| THE HAGUE | 17-05-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document